# EUROPEAN PATENT APPLICATION

(11) **EP 4 268 900 A2**
(43) Date of publication of application: **01.11.2023**
(21) Application number: 23190328.7
(22) Date of filing: 30.05.2018
(51) Int. Cl.: A61P 35/00

(54) **TREATMENT PARADIGM FOR AN ANTI-CD19 ANTIBODY AND VENETOCLAX COMBINATION TREATMENT**

(30) Priority: 31.05.2017 EP 17173712
(62) Divisional of application: 18728153.0
(71) Applicant: MorphoSys AG, 82152 Planegg (DE)
(72) Inventor: KELEMEN, Peter, 82152 Planegg (DE); SCHWARZ, Michael, 82152 Planegg (DE); WINDERLICH, Mark, 82152 Planegg (DE); HEEGER, Steffen, 68165 Mannheim (DE); WEINELT, Dominika, 82152 Planegg (DE)
(74) Representative: Carpmaels & Ransford LLP

(57) **Abstract**

The present disclosure provides anti-CD19 antibodies and venetoclax for use in the treatment of non-Hodgkin's lymphoma, chronic lymphocytic leukemia and/or small lymphocytic lymphoma. The anti-CD19 antibodies, in particular MOR00208, and venetoclax are administered to patients suffering non-Hodgkin's lymphoma (NHL), chronic lymphocytic leukemia (CLL) and/or small lymphocytic lymphoma (SLL) according to a specific treatment paradigm to mitigate therapy associated tumor lysis syndrome.

## Description

### Field of the Invention

The present disclosure provides a treatment paradigm comprising anti-CD19 antibodies and venetoclax for use in the treatment of non-Hodgkin's lymphoma, chronic lymphocytic leukemia and/or small lymphocytic lymphoma. The anti-CD19 antibodies, in particular MOR00208, and venetoclax are administered to patients suffering non-Hodgkin's lymphoma (NHL), chronic lymphocytic leukemia (CLL) and/or small lymphocytic lymphoma (SLL) according to a specific treatment paradigm to mitigate therapy associated tumor lysis syndrome.

### Background

B cells are lymphocytes that play a large role in the humoral immune response. They are produced in the bone marrow of most mammals, and represent 5-15% of the circulating lymphoid pool. The principal function of B cells is to make antibodies against various antigens, and are an essential component of the adaptive immune system. Because of their critical role in regulating the immune system, dysregulation of B cells is associated with a variety of disorders, such as lymphomas, and leukemia. These include non-Hodgkin's lymphoma (NHL), chronic lymphocytic leukemia (CLL) and small lymphocytic lymphoma (SSL).

NHL is a heterogeneous malignancy originating from lymphocytes. In the United States (U.S.), the incidence is estimated at 65,000/year with mortality of approximately 20,000 (American Cancer Society, 2006; and SEER Cancer Statistics Review). The disease can occur in all ages, the usual onset begins in adults over 40 years, with the incidence increasing with age. NHL is characterized by a clonal proliferation of lymphocytes that accumulate in the lymph nodes, blood, bone marrow and spleen, although any major organ may be involved. The current classification system used by pathologists and clinicians is the World Health Organization (WHO) Classification of Tumors, which organizes NHL into precursor and mature B-cell or T-cell neoplasms. The PDQ is currently dividing NHL as indolent or aggressive for entry into clinical trials. The indolent NHL group is comprised primarily of follicular subtypes, small lymphocytic lymphoma, MALT (mucosa-associated lymphoid tissue), and marginal zone; indolent encompasses approximately 50% of newly diagnosed B-cell NHL patients. Aggressive NHL includes patients with histologic diagnoses of primarily diffuse large B cell (DLBL, DLBCL, or DLCL) (40% of all newly diagnosed patients have diffuse large cell), Burkitt's, and mantle cell.

In addition to NHL there are several types of leukemia that result from dysregulation of B cells.

Chronic lymphocytic leukemia (also known as "chronic lymphoid leukemia" or "CLL"), is a type of adult leukemia caused by an abnormal accumulation of B lymphocytes. In CLL, the malignant lymphocytes may look normal and mature, but they are not able to cope effectively with infection. CLL is the most common form of leukemia in adults. Men are twice as likely to develop CLL as women. However, the key risk factor is age. Over 75% of new cases are diagnosed in patients over age 50. More than 10,000 cases are diagnosed every year and the mortality is almost 5,000 a year (American Cancer Society, 2006; and SEER Cancer Statistics Review). CLL is an incurable disease but progresses slowly in most cases. Many people with CLL lead normal and active lives for many years. Because of its slow onset, early-stage CLL is generally not treated since it is believed that early CLL intervention does not improve survival time or quality of life. Instead, the condition is monitored over time. Initial CLL treatments vary depending on the exact diagnosis and the progression of the disease. There are dozens of agents used for CLL therapy. Combination chemotherapy regimens such as FCR (fludarabine, cyclophosphamide and rituximab), and BR (Ibrutinib and rituximab) are effective in both newlydiagnosed and relapsed CLL. Allogeneic bone marrow (stem cell) transplantation is rarely used as a first-line treatment for CLL due to its risk.

Another type of leukemia is Small lymphocytic lymphoma (SLL) that is considered a CLL variant that lacks the clonal lymphocytosis required for the CLL diagnosis, but otherwise shares pathological and immunophenotypic features (Campo et al., 2011). The definition of SLL requires the presence of lymphadenopathy and/or splenomegaly. Moreover, the number of B lymphocytes in the peripheral blood should not exceed 5 × 109/L. In SLL, the diagnosis should be confirmed by histopathologic evaluation of a lymph node biopsy whenever possible (Hallek et al., 2008). The incidence of SLL is approximately 25% of CLL in the US (Dores et al., 2007).

The human CD19 molecule is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD 19 is expressed by most pre-B acute lymphoblastic leukemias (ALL), non-Hodgkin's lymphomas, B cell chronic lymphocytic leukemias (CLL), small lymphocytic lymphomas (SLL), pro-lymphocytic leukemias, hairy cell leukemias, common acute lymphocytic leukemias, and some Null-acute lymphoblastic leukemias (Nadler et al, J. Immunol., 131 :244-250 (1983), Loken et al, Blood, 70:1316-1324 (1987), Uckun et al, Blood, 71 :13- 29 (1988), Anderson et al, 1984. Blood, 63:1424-1433 (1984), Scheuermann, Leuk. Lymphoma, 18:385-397(1995)). The expression of CD 19 on plasma cells further suggests it may be expressed on differentiated B cell tumors such as multiple myeloma, plasmacytomas, Waldenstrom's tumors (Grossbard et al., Br. J. Haematol, 102:509- 15(1998); Treon et al, Semin. Oncol, 30:248-52(2003)).

Therefore, the CD19 antigen is a target for immunotherapy in the treatment of non-Hodgkin's lymphoma, chronic lymphocytic leukemia and/or small lymphocytic lymphoma, including each of the subtypes described herein.

MOR00208 (previously named XmAb5574) is an Fc engineered humanized monoclonal antibody that binds CD19. The increase in binding of MOR00208 Fc to FcyR, due to XmAb engineered mutations, significantly enhances in-vitro antibody dependent cell-mediated cytotoxicity (ADCC), antibody dependent cell-mediated phagocytosis (ADCP), and direct cytotoxic effects (apoptosis) on tumor relative to the unmodified antibody. MOR00208 has not been shown to mediate complement dependent cytotoxicity. In previous studies MOR00208 was combined with fludarabine, bendamustine, idelalisib and also venetoclax. Synergistic activities were observed for the individual combination partners of MOR00208 (WO2013/24097, WO2013/024095, WO2017/032679 and WO2018/078123). Furthermore chimeric antigen receptor (CAR) T cell targeting CD19 and ABT-737, a BCL-2 inhibitor, navitoclax where used in combination to treat B-cell malignancies (Karlsson et al, Cancer Gene Therapy, vol. 20, no, 7, 2013-06.21, pages 386-393)
MOR00208 has or is currently being studied in several clinical trials in CLL, ALL and NHL. In a current Phase II trial (COSMOS) the Efficacy and Safety of MOR00208 plus venetoclax in Patients with Relapsed or Refractory CLL, SLL is studied. Venetoclax, also known as GDC-0199, ABT-199, and RG7601 is a BCL-2 inhibitor indicated for the treatment of patients with chronic lymphocytic leukemia (CLL) with 17p deletion, as detected by an FDA approved test, who have received at least one prior therapy. Venetoclax is described in US Patent Nos. 8,546,399 and 9,174,982, which are all incorporated by reference in their entireties.

However, treatment with anti-CD19 antibodies and also the treatment with venetoclax are not without its side effects. Tumor Lysis Syndrome (TLS) is the major risk of treatment with venetoclax. TLS and relevant laboratory abnormalities were reported in 6% of CLL patients (N=66) with recommended risk stratification, dosing regimen and prophylaxis. Also under treatment with MOR00208 five patients (4%) developed TLS so far. Three in the ALL indication (14%), one in NHL (1%) and one in CLL (4%).

Tumor lysis syndrome (TLS) is a metabolic syndrome that is caused by the sudden killing of tumor cells with anti-tumor cell killing, and subsequent release of cellular contents with the release of large amounts of potassium, phosphate, and nucleic acids into the systemic circulation. Catabolism of the nucleic acids to uric acid leads to hyperuricemia; the marked increase in uric acid excretion can result in the precipitation of uric acid in the renal tubules and renal vasoconstriction, impaired autoregulation, decreased renal flow, oxidation, and inflammation, resulting in acute kidney injury. Hyperphosphatemia with calcium phosphate deposition in the renal tubules can also cause acute kidney injury. High concentrations of both uric acid and phosphate potentiate the risk of acute kidney injury because uric acid precipitates more readily in the presence of calcium phosphate and vice versa that results in hyperkalemia, hyperphosphotemia, hypocalcemia, remia, and acute renal failure. It usually occurs in patients with bulky, rapidly proliferating, treatment-responsive tumors (Jagasia et al., Complications of hematopoietic neoplasms. Wintrobe MM, Greer JP, Foerster J, et al. Wintrobe's Clinical Hematology. 11th ed. Philadelphia, Pa: Lippincott Williams &Wilkins; 2003. Vol II: 1919-44).

However the therapy based on the combination of MOR00208 and venetoclax provides a promising therapeutic approach for use in the treatment of CLL, NHL and/or ALL but potentially bears an increased risk for TLS based on the additive/overlapping cytotoxicities of MOR00208 and venetoclax.

Although most adverse events are tolerable and acceptable, it is a permanent aim to identify the best tolerable dosing of such therapeutics to mitigate side effects as TLS. Accordingly, it is an object of the present disclosure to provide a treatment paradigm for the combination of MOR00208 and venetoclax which reduces the risk of an adverse inflammatory response, such as TLS, and which can be administered without adversely affecting efficacy of the MOR00208 and venetoclax combination treatment.

### Summary

The present disclosure for the first time provides a treatment paradigm for a combination therapy comprising an anti-CD19 antibody, in particular MOR00208, and a BCL-2 inhibitor, in particular venetoclax, which mitigates the risk of therapy associated TLS syndrome.

The new treatment translates the combination treatment into a better safety profile without a delay in the provision of care for patients in need. In order to prevent an increased TLS risk by the concomitant use of both drugs, the treatment with MOR00208 will start one week before the start of the ramp-up treatment with venetoclax at the lowest dose of 20 mg. The MOR00208 administration at the first week (initial dose on Day 1 and the loading dose on Day 4) significantly decreases the absolute lymphocyte count (ALC) close or even below to the critical threshold of 25×10⁹/L for tumor burden assessment described in the Prescribing Information of Venclexta^{®}, hereby mitigating the risk for TLS for subsequent combination treatment of MOR00208 with venetoclax during weekly ramp-up phase of venetoclax.

In accordance with the Venclexta^{®} Prescribing Information, the venetoclax dose is administered according to a weekly ramp-up schedule over 5 weeks to the recommended daily dose of 400 mg. The 5-week ramp-up dosing schedule, starting on Day 8, is designed to gradually reduce tumor burden (debulk) thus decreasing the risk to develop a tumor lysis syndrome (TLS).

In one aspect the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered at least 7 days prior to the first administration of said BCL-2 inhibitor.

In another aspect the present disclosure provides the use of a combination comprising an anti-CD19 antibody and a BCL-2 inhibitor in the treatment of a patient suffering from non-Hodgkin's lymphoma, chronic lymphocytic leukemia and/or small lymphocytic lymphoma, wherein said anti-CD19 antibody is administered at least 7 days prior to the first administration of said BCL-2 inhibitor.

In another aspect the present disclosure provides the use of a combination comprising an anti-CD19 antibody and a BCL-2 inhibitor in the manufacture of a medicament for use in the treatment of a patient suffering from non-Hodgkin's lymphoma, chronic lymphocytic leukemia and/or small lymphocytic lymphoma, wherein said anti-CD19 antibody is administered at least 7 days prior to the first administration of said BCL-2 inhibitor.

In another aspect the present disclosure provides a method for the treatment of non-Hodgkin's lymphoma, chronic lymphocytic leukemia and/or small lymphocytic lymphoma in a patient, wherein the method comprises the administration of a combination comprising an anti-CD19 antibody and a BCL-2 inhibitor wherein said anti-CD19 antibody is administered at least 7 days prior to the first administration of said BCL-2 inhibitor.
In one embodiment the patient is a human patient

In one embodiment said anti-CD19 antibody and said BCL-2 inhibitor are administered separately. In one embodiment said anti-CD19 antibody is administered at least 7 days prior to the first administration of said BCL-2 inhibitor. In another embodiment said anti-CD19 antibody is administered at least 8, 9, 10, 11, 12, 13, 14, 15, 16, 17, 18, 19, 20 or 21 days prior to the first administration of said BCL-2 inhibitor.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered weekly, bi-weekly or monthly after a first administration on Day 1 and wherein the BCL-2 inhibitor is administered for the first time on Day 8 with a starting dose for a weekly ramp-up. In a further embodiment, the anti-CD19 antibody after a first administration on Day 1 is administered weekly for the first 3 months. In a further embodiment the anti-CD19 antibody after a first administration on Day 1 is administered weekly for the first 3 months and bi-weekly for at least the next 3 months. In another embodiment the anti-CD19 antibody after a first administration on Day 1 is administered weekly for the first 3 months, bi-weekly for the next 3 months and monthly thereafter. In yet another embodiment the anti-CD19 antibody after a first administration on Day 1 is administered weekly for the first 3 months, bi-weekly for the next 3 months and monthly thereafter with an additional dose on Day 4. In a further embodiment there is an additional dosing of the anti-CD19 antibody on Day 4.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered weekly, bi-weekly or monthly after a first administration on Day 1 and wherein said BCL-2 inhibitor is administered for the first time on Day 8 with a starting dose of 20mg followed by a weekly ramp-up dosing of 50mg, 100mg, 200mg and 400mg. In another embodiment the BCL-2 inhibitor is administered for the first time on Day 8 with a starting dose of 20mg followed by a weekly ramp-up dosing of 50mg, 100mg, 200mg and 400mg followed by a daily dosing of 400mg.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody after a first administration on Day 1 is administered weekly for the first 3 months, bi-weekly for the next 3 months and monthly thereafter and wherein said BCL-2 inhibitor is administered for the first time on Day 8 with a starting dose of 20mg followed by a weekly ramp-up dosing of 50mg, 100mg, 200mg and 400mg. In another embodiment the BCL-2 inhibitor is administered for the first time on Day 8 with a starting dose of 20mg followed by a weekly ramp-up dosing of 50mg, 100mg, 200mg and 400mg followed by a daily dosing of 400mg.

In one embodiment said BCL-2 inhibitor is venetoclax.

In one embodiment said anti-CD19 antibody comprises an HCDR1 region comprising sequence SYVMH (SEQ ID NO: 1), an HCDR2 region comprising sequence NPYNDG (SEQ ID NO: 2), an HCDR3 region comprising sequence GTYYYGTRVFDY (SEQ ID NO: 3), an LCDR1 region comprising sequence RSSKSLQNVNGNTYLY (SEQ ID NO: 4), an LCDR2 region comprising sequence RMSNLNS (SEQ ID NO: 5), and an LCDR3 region comprising sequence MQHLEYPIT (SEQ ID NO: 6).

In another embodiment said anti-CD19 antibody comprises a variable heavy chain comprising the sequence

EVQLVESGGGLVKPGGSLKLSCAASGYTFTSYVMHWVRQAPGKGLEWIGYINPYNDGTKY
NEKFQGRVTISSDKSISTAYMELSSLRSEDTAMYYCARGTYYYGTRVFDYWGQGTLVTVSS
(SEQ ID NO: 10) and a variable light chain of the sequence

DIVMTQSPATLSLSPGERATLSCRSSKSLQNVGNTYLYWFQQKPGQSPQLLIYRMSNLNS
GVPDRFSGSGSGTEFTLTISSLEPEDFAVYYCMQHLEYPITFGAGTKLEIK (SEQ ID NO: 11).

In another embodiment said anti-CD19 antibody comprises a heavy chain constant domain comprising the sequence

ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPDV
FLFPPKPKDTLMISRTPEVTCVVVDVSHEDPEVQFNWYVDGVEVHNAKTKPREEQFNSTFR
VVSVLTVVHQDWLNGKEYKCKVSNKALPAPEEKTISKTKGQPREPQVYTLPPSREEMTKNQ
VSLTCLVKGFYPSDIAVEWESNGQPENNYKTTPPMLDSDGSFFLYSKLTVDKSRWQQGNV
FSCSVMHEALHNHYTQKSLSLSPGK (SEQ ID NO: 12).

In another embodiment said anti-CD19 antibody comprises a light chain constant domain comprising the sequence


. (SEQ ID NO: 13)

In another embodiment said anti-CD19 antibody comprises a heavy chain constant domain comprising the sequence 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPDV
FLFPPKPKDTLMISRTPEVTCVVVDVSHEDPEVQFNWYVDGVEVHNAKTKPREEQFNSTFR
VVSVLTVVHQDWLNGKEYKCKVSNKALPAPEEKTISKTKGQPREPQVYTLPPSREEMTKNQ
VSLTCLVKGFYPSDIAVEWESNGQPENNYKTTPPMLDSDGSFFLYSKLTVDKSRWQQGNV
FSCSVMHEALHNHYTQKSLSLSPGK (SEQ ID NO: 12) and a light chain constant domain
 comprising the sequence


. (SEQ ID NO: 13)

In another embodiment said anti-CD19 antibody is administered in a concentration of 12mg/kg.

In another embodiment the present disclosure provides a combination for use in the treatment of chronic lymphocytic leukemia, small lymphocytic lymphoma or non-Hodgkin's lymphoma, wherein the non-Hodgkin's lymphoma is selected from the group consisting of follicular lymphoma, small lymphocytic lymphoma, mucosa-associated lymphoid tissue, marginal zone, diffuse large B cell, Burkitt's, and mantle cell.

### Description of Drawings

**Figure 1** illustrates the venetoclax ramp-up.
**Figure 2** shows the amino acid sequence of the variable domains of MOR00208.
**Figure 3** shows the amino acid sequence of the Fc regions of MOR00208.

### Detailed description of the invention

The term "**antibody**" means monoclonal antibodies, including any isotype, such as, IgG, IgM, IgA, IgD and IgE. An IgG antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are referred to as CDR1, CDR2, and CDR3, numbered sequentially from the N-terminus. The more highly conserved portions of the variable regions outside of the CDRs are called the "framework regions". An "antibody fragment" means an Fv, scFv, dsFv, Fab, Fab' F(ab')2 fragment, or other fragment, which contains at least one variable heavy or variable light chain, each containing CDRs and framework regions.

Bcl-2 (B-cell lymphoma 2), encoded in humans by the BCL2 gene, is the founding member of the Bcl-2 family of regulator proteins that regulate cell death (apoptosis), by either inducing (pro-apoptotic) or inhibiting (anti-apoptotic) apoptosis. Bcl-2 is referenced by the NCBI Gene number 596. Bcl-2 is specifically considered an important anti-apoptotic protein and is thus classified as an oncogene. Bcl-2 derives its name from B-cell lymphoma 2, as it is the second member of a range of proteins initially described in chromosomal translocations involving chromosomes 14 and 18 in follicular lymphomas. Orthologs (such as Bcl2 in mice) have been identified in numerous mammals for which complete genome data are available.

A "**BCL-2 inhibitor**" is a class of drug that functions by inhibiting anti-apoptotic B-cell lymphoma-2 (Bcl-2) protein, leading to programmed cell death of cells. BCL-2 inhibitor include venetoclax. Venetoclax is marketed by Abbvie and Genentech (trade name VENCLEXTA^{™}, also known as GDC-0199, ABT-199, and RG7601). Venetoclax is currently labelled for the treatment of patients with chronic lymphocytic leukemia (CLL) with 17p deletion, as detected by an FDA approved test, who have received at least one prior therapy. The formula of venetoclax is 4-(4-{[2-(4-Chlorophenyl)-4,4-dimethyl-1-cyclohexen-1-yl]methyl}-1-piperazinyl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide and has the following structure:

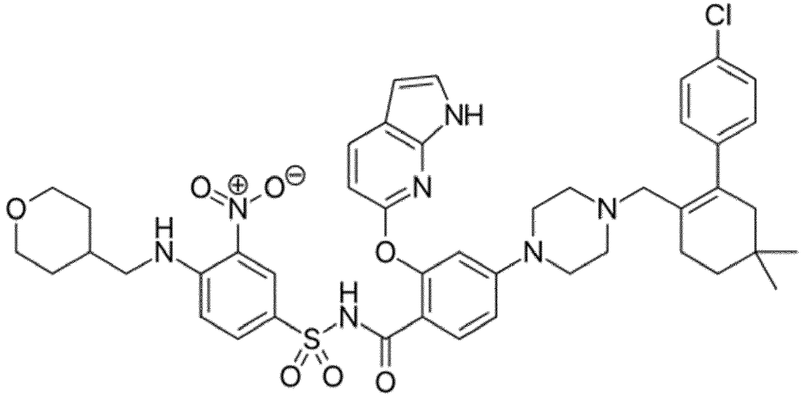

**"Venetoclax,"** "ABT", and "ABT-199" are used as synonyms herein.

Other BCL-2 inhibitors include:
Genasense: An antisense oligonucleotide drug Genasense (G3139) was developed by Genta Incorporated to target Bcl-2. An antisense DNA or RNA strand is non-coding and complementary to the coding strand (which is the template for producing respectively RNA or protein). An antisense drug is a short sequence of RNA that hybridises with and inactivates mRNA, preventing the protein from being formed. Human lymphoma cell proliferation (with t(14;18) translocation) could be inhibited by antisense RNA targeted at the start codon region of Bcl-2 mRNA. In vitro studies led to the identification of Genasense, which is complementary to the first 6 codons of Bcl-2 mRNA. These showed successful results in Phase I/II trials for lymphoma. A large Phase III trial was launched in 2004. As of 2016, the drug had not been approved and its developer was out of business.

ABT-737 and ABT-263: In the mid-2000s, Abbott Laboratories developed a novel inhibitor of Bcl-2, Bcl-xL and Bcl-w, known as ABT-737. This compound is part of a group of BH3 mimetic small molecule inhibitors (SMI) that target these Bcl-2 family proteins, but not A1 or Mcl-1. ABT-737 is superior to previous BCL-2 inhibitors given its higher affinity for Bcl-2, Bcl-xL and Bcl-w. In vitro studies showed that primary cells from patients with B-cell malignancies are sensitive to ABT-737. In animal models, it improves survival, causes tumor regression and cures a high percentage of mice. In preclinical studies utilizing patient xenografts, ABT-737 showed efficacy for treating lymphoma and other blood cancers. Because of its unfavorable pharmacologic properties ABT-737 is not appropriate for clinical trials, while its derivative ABT-263 has similar activity on small cell lung cancer (SCLC) cell lines and has entered clinical trials.

The term "**ramp-up**" used herein means increasing a dose from a first set point to further set points over a predetermined time period.

"**VH**" refers to the variable region of an immunoglobulin heavy chain of an antibody, or antibody fragment. "**VL**" refers to the variable region of the immunoglobulin light chain of an antibody, or antibody fragment.

The term "**CD19**" refers to the protein known as CD19, having the following synonyms: B4, B-lymphocyte antigen CD19, B-lymphocyte surface antigen B4, CVID3, Differentiation antigen CD19, MGC12802, and T-cell surface antigen Leu-12.

Human CD19 has the amino acid sequence of:










. (SEQ ID NO: 7)

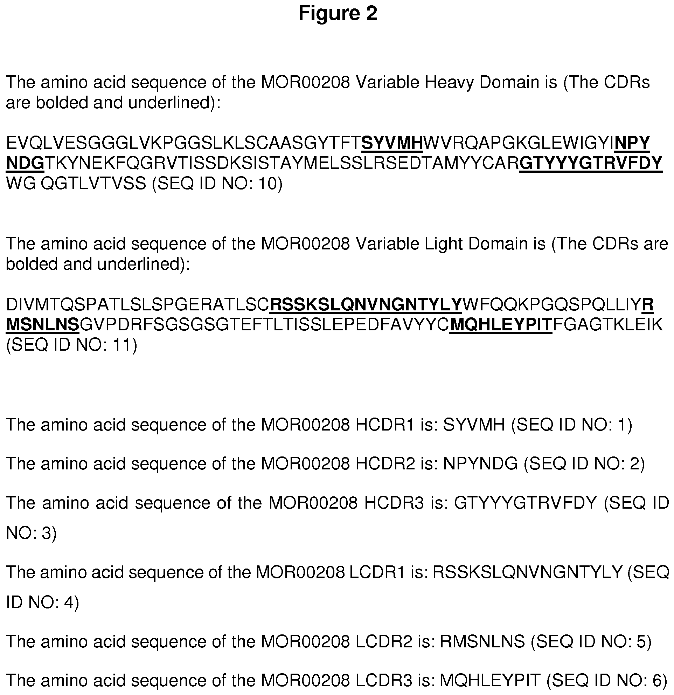

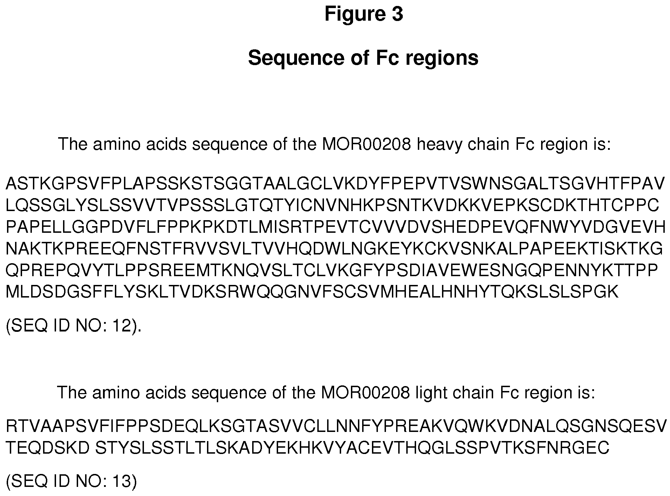

"**MOR00208**" is an anti-CD19 antibody. The amino acid sequence of the variable domains is provided in Figure 2. The amino acid sequence of the heavy and light chain Fc regions of MOR00208 are provided in Figure 3. "MOR00208" and "XmAb 5574" are used as synonyms to describe the antibody shown in Figures 2 and 3. The MOR00208 antibody is described in US patent application serial number 12/377,251, which is incorporated by reference in its entirety and describes the antibody named 4G7 H1.52 Hybrid S239D/I332E/ 4G7 L1.155 (later named MOR00208) as follows:
>4G7 H1.52 Hybrid S239D/I332E 
EVQLVESGGGLVKPGGSLKLSCAASGYTFTSYVMHWVRQAPGKGLEWIGYINPYN
DGTKYNEKFQGRVTISSDKSISTAYMELSSLRSEDTAMYYCARGTYYYGTRVFDYWGQGTL
VTVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPELL
GGPDVFLFPPKPKDTLMISRTPEVTCVVVDVSHEDPEVQFNWYVDGVEVHNAKTKPREEQ
FNSTFRVVSVLTVVHQDWLNGKEYKCKVSNKALPAPEEKTISKTKGQPREPQVYTLPPSRE
EMTKNQVSLTCLVKGFYPSDIAVEWESNGQPENNYKTTPPMLDSDGSFFLYSKLTVDKSR
WQQGNVFSCSVMHEALHNHYTQKSLSLSPGK (SEQ ID NO: 14)
> 4G7 L1.155

DIVMTQSPATLSLSPGERATLSCRSSKSLQNVNGNTYLYWFQQKPGQSPQLLIYRM
SNLNSGVPDRFSGSGSGTEFTLTISSLEPEDFAVYYCMQHLEYPITFGAGTKLEIKRTVAAPS
VFIFPPSDEQLKSGTASVVCLLNNFYPREAKVQWKVDNALQSGNSQESVTEQDSKDSTYSL
SSTLTLSK ADYEKHKVYACEVTHQGLSSPVTKSFNRGEC (SEQ ID NO: 15)

Additional antibodies specific for CD19 are described in US patent no. 7,109,304 (Immunomedics), which is incorporated by reference in its entirety; US application serial no. 11/917,750 (Medarex), which is incorporated by reference in its entirety; US application serial no. 11/852,106 (Medimmune), which is incorporated by reference in its entirety; US application serial no. 11/648,505 (Merck Patent GmbH), which is incorporated by reference in its entirety; US patent no. 7,968,687 (Seattle Genetics), which is incorporated by reference in its entirety; and US application serial no. 12/710,442 (Glenmark Pharmaceuticals), which is incorporated by reference in its entirety.

"**Fc region"** means the constant region of an antibody, which in humans may be of the IgG1, 2, 3, 4 subclass or others. The sequences of human Fc regions are available at the IMGT website.

A **"combination"** means more than one item, e.g. a compound such as an antibody and venetoclax.

The present disclosure also relates to combinations, pharmaceuticals, and pharmaceutical compositions containing the described combinations. The two components of the combination of the present invention, e.g. the antibody specific for CD19 and venetoclax, may be administered together, simultaneously, separately or subsequently, either physically or in time.

Venetoclax is currently taken orally and is currently dosed once per day. MOR00208 is currently administered intravenously, and is currently dosed either once a week ("**weekly**") or once every two weeks ("**bi-weekly**") or once a month ("**monthly**).

Preferably, administration of both drugs allows for both drugs to be active in the patient at the same time. For example, if MOR00208 is dosed weekly and venetoclax is dosed daily then the active substance of both drugs is present in the patient at the same time. In an embodiment, MOR00208, is administered prior to and/or separately from the administration venetoclax.

Simultaneously means that the two components are administered at a time where both components (drugs) are active in the patient at the same time.

Administered together can mean administered at the same time.

The components of the combination may be formulated in different pharmaceutical compositions. A pharmaceutical composition includes an active agent, e.g. an antibody for therapeutic use in humans. A pharmaceutical composition may include acceptable carriers or excipients.

"**Administered**" or "**administration**" includes but is not limited to delivery by an injectable form, such as, for example, an intravenous, intramuscular, intradermal or subcutaneous route or mucosal route, for example, as a nasal spray or aerosol for inhalation or as an ingestable solution, capsule or tablet.

A "**therapeutically effective amount**" of a compound or combination refers to an amount sufficient to cure, alleviate or partially arrest the clinical manifestations of a given disease or disorder and its complications. The amount that is effective for a particular therapeutic purpose will depend on the severity of the disease or injury as well as on the weight and general state of the subject. It is understood that determination of an appropriate dosage may be achieved, using routine experimentation, by constructing a matrix of values and testing different points in the matrix, all of which is within the ordinary skills of a trained physician or clinical scientist.

### Embodiments

As the mechanism of action of venetoclax and other BCL-2 inhibitors are similar, it is believed that the dosing paradigm as disclosed herein should also be effective and mitigate the risk of TLS when treating humans having non-Hodgkin's lymphoma, chronic lymphocytic leukemia and/or small lymphocytic lymphoma with a combination of the exemplified anti-CD19 antibody and a BCL-2 inhibitor other than venetoclax.

As the exemplified anti-CD19 antibody and other anti-CD19 antibodies bind CD19, it is believed that the dosing paradigm as disclosed herein should also be effective and mitigate the risk of TLS when treating humans having non-Hodgkin's lymphoma, chronic lymphocytic leukemia and/or small lymphocytic lymphoma with a combination of any anti-CD19 antibody and a B-cell lymphoma-2 (Bcl-2) protein inhibitor, where the anti-CD19 antibody is, for example, described in US patent application serial number 12/377,251 (Xencor), WO2005012493, WO2010053716 (Immunomedics); WO2007002223 (Medarex); WO2008022152 (Xencor); WO2008031056 (Medimmune); WO 2007/076950 (Merck Patent GmbH); WO 2009/052431 (Seattle Genetics); and WO2010095031 (Glenmark Pharmaceuticals), all of which are incorporated by reference in their entireties.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered weekly, bi-weekly or monthly after a first administration on Day 1 and wherein the BCL-2 inhibitor is administered for the first time on Day 8 with a starting dose for a weekly ramp-up.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered weekly, bi-weekly or monthly after a first administration on Day 1 and wherein the BCL-2 inhibitor is administered for the first time on Day 8 with a starting dose for a weekly ramp-up until the daily dose of 400mg.

In a further embodiment, the anti-CD19 antibody after a first administration on Day 1 is administered weekly for the first 3 months. In a further embodiment the anti-CD19 antibody after a first administration on Day 1 is administered weekly for the first 3 months and bi-weekly for at least the next 3 months. In another embodiment the anti-CD19 antibody after a first administration on Day 1 is administered weekly for the first 3 months, bi-weekly for the next 3 months and monthly thereafter. In yet another embodiment the anti-CD19 antibody after a first administration on Day 1 is administered weekly for the first 3 months, bi-weekly for the next 3 months and monthly thereafter with an additional dose on Day 4. In a further embodiment there is an additional dosing of the anti-CD19 antibody on Day 4.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered weekly, bi-weekly or monthly after a first administration on Day 1 and wherein said BCL-2 inhibitor is administered for the first time on Day 8 with a starting dose of 20mg followed by a weekly ramp-up dosing of 50mg, 100mg, 200mg and 400mg. In another embodiment the BCL-2 inhibitor is administered for the first time on Day 8 with a starting dose of 20mg followed by a weekly ramp-up dosing of 50mg, 100mg, 200mg and 400mg followed by a daily dosing of 400mg. In a further embodiment there is an additional dosing of the anti-CD19 antibody on Day 4.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody after a first administration on Day 1 is administered weekly for the first 3 months, bi-weekly for the next 3 months and monthly thereafter and wherein said BCL-2 inhibitor is administered for the first time on Day 8 with a starting dose of 20mg followed by a weekly ramp-up dosing of 50mg, 100mg, 200mg and 400mg. In another embodiment the BCL-2 inhibitor is administered for the first time on Day 8 with a starting dose of 20mg followed by a weekly ramp-up dosing of 50mg, 100mg, 200mg and 400mg followed by a daily dosing of 400mg. In a further embodiment there is an additional dosing of the anti-CD19 antibody on Day 4.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered weekly, bi-weekly or monthly after a first administration on Day 1 and wherein said BCL-2 inhibitor is administered for the first time on Day 8 with a starting dose of 10, 15 or 20mg followed by a weekly ramp-up dosing of 20, 25, 30, 35, 40, 45 or 50 mg, 50, 55, 60, 65, 70, 75, 80, 85 90, 95 or 100mg, 100, 110, 120, 130, 140, 150, 160, 170,180, 190 or 200mg and 200, 250, 300, 350 or 400mg. In another embodiment the BCL-2 inhibitor is administered for the first time on Day 8 with a starting dose of 20mg followed by a weekly ramp-up dosing of 20, 25, 30, 35, 40, 45 or 50 mg, 50, 55, 60, 65, 70, 75, 80, 85 90, 95 or 100mg, 100, 110, 120, 130, 140, 150, 160, 170,180, 190 or 200mg followed by a daily dosing of 200, 250, 300, 350 or 400mg. In a further embodiment there is an additional dosing of the anti-CD19 antibody on Day 4.

In one embodiment the present disclosure provides a combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody after a first administration on Day 1 is administered weekly for the first 3 months, bi-weekly for the next 3 months and monthly thereafter and wherein said BCL-2 inhibitor is administered for the first time on Day 8 with a starting dose of 10, 15 or 20mg followed by a weekly ramp-up dosing of 20, 25, 30, 35, 40, 45 or 50 mg, 50, 55, 60, 65, 70, 75, 80, 85 90, 95 or 100mg, 100, 110, 120, 130, 140, 150, 160, 170,180, 190 or 200mg and 200, 250, 300, 350 or 400mg. In another embodiment the BCL-2 inhibitor is administered for the first time on Day 8 with a starting dose of 20mg followed by a weekly ramp-up dosing of 20, 25, 30, 35, 40, 45 or 50 mg, 50, 55, 60, 65, 70, 75, 80, 85 90, 95 or 100mg, 100, 110, 120, 130, 140, 150, 160, 170,180, 190 or 200mg followed by a daily dosing of 200, 250, 300, 350 or 400mg.

In another embodiment said anti-CD19 antibody is administered in a concentration of 12mg/kg.

In embodiments, the BCL-2 inhibitor is venetoclax.

In embodiments, the components of the combination, the anti-CD19 antibody and venetoclax, are administered separately. In an embodiment, venetoclax is administered prior to administration of the anti-CD19 antibody.

In embodiments, the components of the combination are administered at a time where both components (drugs) are active in the patient at the same time. It is implied by "synergism" that both drugs are active in the patient at the same time. In embodiments, the components of the combination are administered together, simultaneously, separately or subsequently, either physically or in time. In embodiments, the components of the combination are administered simultaneously.

In embodiments the combination is a pharmaceutical composition. In embodiments, the composition comprises an acceptable carrier. In embodiments, the combination is administered in an effective amount.

In embodiments, the non-Hodgkin's lymphoma is follicular lymphoma. In embodiments, the non-Hodgkin's lymphoma is small lymphocytic lymphoma. In embodiments, the non-Hodgkin's lymphoma is mucosa-associated lymphoid tissue. In embodiments, the non-Hodgkin's lymphoma is marginal zone lymphoma. In embodiments, the non-Hodgkin's lymphoma is diffuse large B cell lymphoma. In embodiments, the non-Hodgkin's lymphoma is Burkitt's lymphoma. In embodiments, the non-Hodgkin's lymphoma is mantle cell lymphoma.

In one aspect the present disclosure provides a combination comprising MOR00208 antibody and venetoclax, for use in the treatment of a patient suffering from non-Hodgkin's lymphoma, chronic lymphocytic leukemia and/or small lymphocytic lymphoma, wherein MOR00208 after a first administration on Day 1 is administered weekly for the first 3 months, bi-weekly for the next 3 months and monthly thereafter, with an additional dosing of tMOR00208 on Day 4 and wherein venetoclax is administered for the first time on Day 8 with a starting dose of 20mg followed by a weekly ramp-up dosing of 50mg, 100mg, 200mg and 400mg. In another embodiment venetoclax is administered for the first time on Day 8 with a starting dose of 20mg followed by a weekly ramp-up dosing of 50mg, 100mg, 200mg and 400mg followed by a daily dosing of 400mg. In an embodiment MOR00208 is administered in a concentration of 12mg/kg.

In one aspect the present disclosure provides a combination comprising an anti-CD19 antibody and venetoclax, for use in the treatment of a patient suffering from non-Hodgkin's lymphoma, chronic lymphocytic leukemia and/or small lymphocytic lymphoma, wherein said anti-CD19 antibody after a first administration on Day 1 is administered weekly for the first 3 months, bi-weekly for the next 3 months and monthly thereafter, with an additional dosing on Day 4 and wherein venetoclax is administered for the first time on Day 8 with a starting dose of 20mg followed by a weekly ramp-up dosing of 50mg, 100mg, 200mg and 400mg. In another embodiment venetoclax is administered for the first time on Day 8 with a starting dose of 20mg followed by a weekly ramp-up dosing of 50mg, 100mg, 200mg and 400mg followed by a daily dosing of 400mg.

In one embodiment said anti-CD19 antibody comprises an HCDR1 region comprising sequence SYVMH (SEQ ID NO: 1), an HCDR2 region comprising sequence NPYNDG (SEQ ID NO: 2), an HCDR3 region comprising sequence GTYYYGTRVFDY (SEQ ID NO: 3), an LCDR1 region comprising sequence RSSKSLQNVNGNTYLY (SEQ ID NO: 4), an LCDR2 region comprising sequence RMSNLNS (SEQ ID NO: 5), and an LCDR3 region comprising sequence MQHLEYPIT (SEQ ID NO: 6).

In another embodiment said anti-CD19 antibody comprises a variable heavy chain comprising the sequence 
EVQLVESGGGLVKPGGSLKLSCAASGYTFTSYVMHWVRQAPGKGLEWIGYINPYNDGTKY
NEKFQGRVTISSDKSISTAYMELSSLRSEDTAMYYCARGTYYYGTRVFDYWGQGTLVTVSS
(SEQ ID NO: 10)
 and a variable light chain of the sequence

DIVMTQSPATLSLSPGERATLSCRSSKSLQNVGNTYLYWFQQKPGQSPQLLIYRMSNLNS
GVPDRFSGSGSGTEFTLTISSLEPEDFAVYYCMQHLEYPITFGAGTKLEIK (SEQ ID NO: 11).

In another embodiment said anti-CD19 antibody comprises a heavy chain constant domain comprising the sequence

ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPDV
FLFPPKPKDTLMISRTPEVTCVVVDVSHEDPEVQFNWYVDGVEVHNAKTKPREEQFNSTFR
VVSVLTVVHQDWLNGKEYKCKVSNKALPAPEEKTISKTKGQPREPQVYTLPPSREEMTKNQ
VSLTCLVKGFYPSDIAVEWESNGQPENNYKTTPPMLDSDGSFFLYSKLTVDKSRWQQGNV
FSCSVMHEALHNHYTQKSLSLSPGK (SEQ ID NO: 12).

In another embodiment said anti-CD19 antibody comprises a light chain constant domain comprising the sequence


. (SEQ ID NO: 13)

In another embodiment said anti-CD19 antibody comprises a heavy chain constant domain comprising the sequence 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPDV
FLFPPKPKDTLMISRTPEVTCVVVDVSHEDPEVQFNWYVDGVEVHNAKTKPREEQFNSTFR
VVSVLTVVHQDWLNGKEYKCKVSNKALPAPEEKTISKTKGQPREPQVYTLPPSREEMTKNQ
VSLTCLVKGFYPSDIAVEWESNGQPENNYKTTPPMLDSDGSFFLYSKLTVDKSRWQQGNV
FSCSVMHEALHNHYTQKSLSLSPGK (SEQ ID NO: 12) and a light chain constant domain
 comprising the sequence


. (SEQ ID NO: 13).

In one aspect the present disclosure provides a combination comprising an anti-CD19 antibody and venetoclax, for use in the treatment of a patient suffering from non-Hodgkin's lymphoma, chronic lymphocytic leukemia and/or small lymphocytic lymphoma, wherein said anti-CD19 antibody after a first administration on Day 1 is administered weekly for the first 3 months, bi-weekly for the next 3 months and monthly thereafter, with an additional dosing on Day 4 and wherein venetoclax is administered for the first time on Day 8 with a starting dose of 20mg followed by a weekly ramp-up dosing of 50mg, 100mg, 200mg and 400mg, wherein said anti-CD19 antibody comprises a heavy chain constant domain comprising the sequence 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPDV
FLFPPKPKDTLMISRTPEVTCVVVDVSHEDPEVQFNWYVDGVEVHNAKTKPREEQFNSTFR
VVSVLTVVHQDWLNGKEYKCKVSNKALPAPEEKTISKTKGQPREPQVYTLPPSREEMTKNQ
VSLTCLVKGFYPSDIAVEWESNGQPENNYKTTPPMLDSDGSFFLYSKLTVDKSRWQQGNV
FSCSVMHEALHNHYTQKSLSLSPGK (SEQ ID NO: 12) and a light chain constant domain
 comprising the sequence


. (SEQ ID NO: 13).

In another embodiment venetoclax is administered for the first time on Day 8 with a starting dose of 20mg followed by a weekly ramp-up dosing of 50mg, 100mg, 200mg and 400mg followed by a daily dosing of 400mg.

In one aspect the present disclosure provides a combination comprising an anti-CD19 antibody and venetoclax, for use in the treatment of a patient suffering from non-Hodgkin's lymphoma, chronic lymphocytic leukemia and/or small lymphocytic lymphoma, wherein said anti-CD19 antibody is administered on Day 1, on Day 4, on Day 8 and weekly for the first 3 months, bi-weekly for the next 3 months and monthly thereafter and wherein venetoclax is administered for the first time on Day 8 with a starting dose of 20mg followed by a weekly ramp-up dosing of 50mg, 100mg, 200mg and 400mg. In another embodiment venetoclax is administered for the first time on Day 8 with a starting dose of 20mg followed by a weekly ramp-up dosing of 50mg, 100mg, 200mg and 400mg followed by a daily dosing of 400mg.

In another aspect the present disclosure provides the use of a combination comprising an anti-CD19 antibody and venetoclax, in the treatment of a patient suffering from non-Hodgkin's lymphoma, chronic lymphocytic leukemia and/or small lymphocytic lymphoma, wherein said anti-CD19 antibody after a first administration on Day 1 is administered weekly for the first 3 months, bi-weekly for the next 3 months and monthly thereafter, with an additional dosing on Day 4 and wherein venetoclax is administered for the first time on Day 8 with a starting dose of 20mg followed by a weekly ramp-up dosing of 50mg, 100mg, 200mg and 400mg. In another embodiment venetoclax is administered for the first time on Day 8 with a starting dose of 20mg followed by a weekly ramp-up dosing of 50mg, 100mg, 200mg and 400mg followed by a daily dosing of 400mg.

In another aspect the present disclosure provides the use of a combination comprising an anti-CD19 antibody and venetoclax, in the manufacture of a medicament for use in the treatment of a patient suffering from non-Hodgkin's lymphoma, chronic lymphocytic leukemia and/or small lymphocytic lymphoma, wherein said anti-CD19 antibody after a first administration on Day 1 is administered weekly for the first 3 months, bi-weekly for the next 3 months and monthly thereafter, with an additional dosing on Day 4 and wherein venetoclax is administered for the first time on Day 8 with a starting dose of 20mg followed by a weekly ramp-up dosing of 50mg, 100mg, 200mg and 400mg. In another embodiment venetoclax is administered for the first time on Day 8 with a starting dose of 20mg followed by a weekly ramp-up dosing of 50mg, 100mg, 200mg and 400mg followed by a daily dosing of 400mg.

In another aspect the present disclosure provides a method for the treatment of non-Hodgkin's lymphoma, chronic lymphocytic leukemia and/or small lymphocytic lymphoma in a patient, wherein the method comprises the administration of a combination comprising an anti-CD19 antibody and venetoclax, wherein said anti-CD19 antibody after a first administration on Day 1 is administered weekly for the first 3 months, bi-weekly for the next 3 months and monthly thereafter, with an additional dosing on Day 4 and wherein venetoclax is administered for the first time on Day 8 with a starting dose of 20mg followed by a weekly ramp-up dosing of 50mg, 100mg, 200mg and 400mg. In another embodiment venetoclax is administered for the first time on Day 8 with a starting dose of 20mg followed by a weekly ramp-up dosing of 50mg, 100mg, 200mg and 400mg followed by a daily dosing of 400mg.

### Examples

### The study

This is a multicenter, open-label phase II study of MOR00208 combined with venetoclax for the treatment of adult patients with relapsed/refractory (R/R) CLL or R/R SLL pretreated with a BTK inhibitor as a single agent or as part of a combination therapy. A total of 120 patients are enrolled in Europe and the US. The primary objective of the study is to determine the safety and the efficacy of the combination MOR00208 and venetoclax.

The study will include a safety run-in phase enrolling 10-12 patients per combination treatment. MOR00208 will be administered at a dose of 12.0 mg/kg as intravenous (IV) infusion and venetoclax as 400 mg oral tablets daily dose.

To mitigate the risk for TLS the treatment with venetoclax is initiated after an initial treatment with MOR00208 at 20 mg up from Cycle 1 Day 8 (C1D8) for 7 days, followed by a weekly ramp-up dosing schedule to the recommended daily dose of 400 mg.

The safety run-in phase is concluded with an evaluation of the safety data of all treated patients once 10 patients completed at least 5 weeks of combination treatment. All 10-12 patients will be dosed sequentially, at least one week apart.

The safety evaluation will be performed by an IDMC after the first 10 patients completed at least 5 weeks of combination treatment. The evaluation will be done by the IDMC as already described. Importantly, all patients who participate in the safety run-in phase, will be hospitalized on the day of the first two dose escalations of venetoclax (C1D8 and C1D15).

The criteria for the evaluation of safety of the combination therapy are laid down in the Safety Management Plan of the trial.

The study commences when the outcome of the respective safety run-in phase is known. If safety evaluation results in a recommended dose reduction of venetoclax, the sponsor will reconsider dosing and amend the protocol accordingly.

An interim analysis will be performed for each cohort in the trial in order to check for futility and safety. While the interim analysis is being conducted, no further patients will be enrolled into that respective cohort. These interim analysis will be based on the response assessment and safety data of the enrolled patients up to the timepoint of the interim analysis, which is defined as 3 months after treatment start of the 40th patient.

Once 40 patients are recruited, recruitment is planned to be paused per discussion with the sponsor. Then recruitment will be re-opened after conduct of the interim analysis, upon decision of the sponsor.

During the study, MOR00208 is administered as infusions 12.0 mg/kg in 28-day cycles. From Cycle 1 to 3, each cycle consists of weekly infusions (on Day 1, Day 8, Day 15 and Day 22). In Cycle 1, an additional loading dose is administered on Day 4. From Cycle 4 to 6, MOR00208 is administered on a biweekly basis with infusions on Day 1 and Day 15 of each cycle. Thereafter, MOR00208 is administered on Day 1 of each cycle starting with Cycle 7 Day 1.

Venetoclax is self-administered starting from Cycle1 Day8 and on Day1 of each cycle onwards. Treatment with venetoclax may be modified in a de-escalating fashion or discontinued based upon clinical and laboratory findings. In case venetoclax treatment was stopped due to side effects suspected to be related to venetoclax, the patient may continue MOR00208 treatment.

Tumor assessments used for the determination of the primary endpoint (ORR) and secondary efficacy outcomes are evaluated through central review of radiological and clinical data by an Independent Radiology/Clinical Review Committee (IRC).

Progressive disease will require discontinuation of study medication. Patients who are discontinued from treatment due to progressive disease will be followed up for survival every 3 months for up to 3 years after EOT. Patients who discontinue from treatment for reasons other than progressive disease or withdrawal of consent will undergo tumor assessment until PD occurs. Then, they will be followed for survival every 3 months.

Patients who benefit from treatment are allowed to continue MOR00208 treatment beyond Cycle 24 at Investigator's discretion. The total duration of the study is limited to 5 years from first patient first visit. Depending on the time of enrolment relative to study initiation, the duration of study participation is different for each patient.

### Patient Inclusion Criteria

Diagnosis/Trial Population
1. Age ≥18 years
2. Chronic lymphocytic leukemia (CLL) or small lymphocytic lymphoma (SLL):
   a) history of diagnosis of CLL or SLL that meets IWCLL diagnostic criteria
   b) histologically confirmed diagnosis of SLL by lymph node biopsy and documented within medical records
   c) indication for treatment as defined by the IWCLL guidelines
3. Patients must have both of the following:
   a) relapsed' or refractory² disease while receiving a BTK inhibitor (e.g., ibrutinib) or intolerance of such therapy
   b) single-agent or combination therapy with a BTK inhibitor for at least one month must be the patient's most recent prior anticancer therapy
      ¹relapsed disease is defined as progressive disease in subjects who have previously achieved a PR or CR to most recent BTK inhibitor therapy
      ²refractory disease is defined as progressive disease in subjects who have previously not achieved a PR or CR to most recent BTK inhibitor therapy, or stable disease as best response after 12 months of receiving the most recent BTK inhibitor therapy
4. Eastern Cooperative Oncology Group (ECOG) performance status of 0 to 2
5. Patients with a past medical history of autologous or allogeneic stem cell transplantation must exhibit full hematological recovery without any evidence of active graft versus host disease before enrolment into the study.
6. Patients must meet the following laboratory criteria at screening:
   - Adequate bone marrow function as follows:
      a) absolute neutrophil count (ANC) ≥ 1.0 × 10⁹/L
      b) platelet count ≥ 30 × 10⁹/L in the absence of clinically significant evidence of bleeding
      c) hemoglobin ≥ 8.0 g/dL
   - Adequate hepatic and renal function as follows:
      d) total serum bilirubin ≦ 1.5 × ULN or ≦ 3 × ULN in cases of documented liver involvement by CLL (For patients with Gilbert's disease, serum bilirubin up to 3 × ULN is allowed provided normal direct bilirubin.)
      e) ALT and AST ≦ 2.5 × ULN or <3 ×ULN in cases of documented liver involvement by CLL
      f) serum creatinine clearance calculated using a standard Cockcroft-Gault formula must be: ≥ 50 mL/min

Other Inclusion Criteria
7. Females of childbearing potential (FCBP; see Appendix F) must:
   a) not be pregnant as confirmed by a negative serum pregnancy test at screening and a urine pregnancy test prior to starting study therapy
   b) refrain from breastfeeding and donating blood or oocytes during the course of the study and for 3 months after the last dose of study medication (Note: All female patients are prohibited to donate blood within the before mentioned time frame)
   c) agree to ongoing pregnancy testing during the course of the study, and until 3 months after study therapy has ended. This applies even if the patient practices complete and continued sexual abstinence
   d) commit to continued abstinence from heterosexual intercourse if it is in accordance with her lifestyle or agree to use and be able to comply with the use of highly effective contraception without interruption during the study and for 3 months after the last dose of study medication
   e) FCBP using hormonal contraceptives should add a barrier method as a second form of contraception since it is currently unknown whether idelalisib or venetoclax may affect the effectiveness of hormonal contraceptives
8. Males must use a highly effective method of contraception without interruption, if the patient is sexually active with a FCBP, refrain from donating blood or sperm during the study participation and for 3 months after the last dose of study medication

### Study Treatments

In this open-label study the treatment consisting of MOR00208 and venetoclax is as scheduled, until disease progression, unacceptable toxicity, or discontinuation for any other reason, whichever comes first.

Each Investigator is responsible for ensuring that deliveries of study drugs and other study materials from the sponsor and other drugs from the appropriate suppliers are correctly received, recorded, handled and stored safely and properly in accordance with all applicable regulatory guidelines, and used in accordance with this protocol. The Investigator can delegate these tasks to another person (according to the local laws and regulations). Drug accountability forms are kept by each site participating in the study and checked during monitoring visits. All dosages prescribed to the patient and all dose changes during the study must be recorded in the eCRF.

Study drug must be handled and/or prepared as described in the Study Drug Preparation Manual, the Investigator Brochure (IB) for MOR00208, and other safety-relevant documents (e.g., the Prescribing Information by US FDA for venetoclax).

### MOR00208

MOR00208 drug product (DP) must be stored under refrigeration at 2 to 8°C in its original package in an appropriate storage facility accessible only to the pharmacist(s), the Investigator, or a duly designated person.

MOR00208 DP is a yellowish lyophilisate supplied in single-use 20 mL glass vials. Each vial contains 200 mg of MOR00208 for reconstitution with 5 mL water for injection (WFI). Reconstitution yields 40 mg/mL MOR00208 in 25 mM sodium citrate, 200 mM trehalose and 0.02% (w/v) polysorbate 20 at pH 6.0. Each product vial is intended to deliver 200 mg of MOR00208 in 5 mL of reconstituted solution. The solution after reconstitution is colorless to slightly yellow and essentially free of foreign particles; it may contain a few white to whitish product-related particles.

For administration, MOR00208 is diluted into a commercially available 250 mL infusion container with 0.9% (w/v) sodium chloride for injection.

MOR00208 is administered IV at a dose of 12.0 mg/kg. For the first 3 months (3 cycles) of the study, each cycle will consist of a MOR00208 infusion on Day 1, Day 8, Day 15 and Day 22 of the cycle. In addition, a loading dose is administered on Day 4 of Cycle 1. Thereafter, MOR00208 is administered on a biweekly basis (every 14 days) with infusions on Day 1 and Day 15 of each cycle from Cycle 4 to 6, and on a monthly basis on Day 1 of each cycle from Cycle 7 Day 1.

The individual MOR00208 infusion is prepared under aseptic conditions and administered at the study site, according to the directions of the sponsor, which is provided in a Study Drug Preparation Manual. In general, a vial of MOR00208 must be used as soon as possible after reconstitution with WFI. Any solution remaining in the vial has to be discarded. After dilution for infusion, administration of MOR00208 should take place as soon as possible.

For the first infusion, the intravenous (IV) infusion rate should be 70 mUh for the first 30 minutes and subsequently increased to a rate of 125 mUh; the total infusion duration should ideally not exceed 2.5 hours. All subsequent MOR00208 infusions are administered IV at a constant rate of 125 mUh over an approximately 2-hour period. MOR00208 should not be administered as an IV push or bolus.

### Venetoclax

Venetoclax tablets as commercially available for oral administration are supplied as pale yellow film-coated tablets that contain 10 or 100 mg venetoclax as the active ingredient or beige film-coated tablets that contain 50 mg venetoclax as the active ingredient. They are to be stored at or below 30°C (86°F).

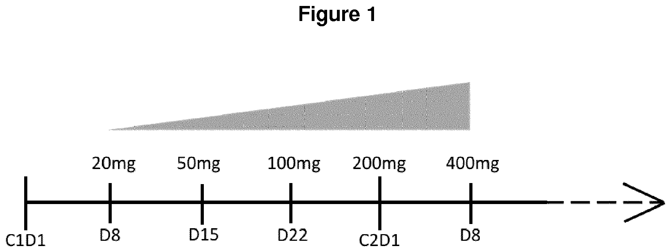

Venetoclax dose should be administered according to a weekly ramp-up schedule over 5 weeks to the recommended daily dose of 400 mg. Figure 1Error! Reference source not **found,** illustrates this initial ramp-up. Dosing schedule for ramp-up phase as follows, daily dose of venetoclax: 20 mg 1^{st} week of combination treatment, 50 mg 2^{nd} week, 100 mg 3^{rd} week, 200 mg 4^{th} week and 400 mg 5^{th} week and thereafter. The 5-week ramp-up dosing schedule, starting on C1 D8, is designed to gradually reduce tumor burden (debulk) and decrease the risk of tumor lysis syndrome (TLS). Assessment of patient-specific factors for level of risk of TLS and prophylaxis for TLS should be performed prior to first dose of venetoclax.

The first 4 weeks of venetoclax treatment are supplied in blisters of respective venetoclax tablets, according to the ramp-up schedule. Once the ramp-up phase is completed, the 400 mg dose is achieved using 100 mg tablets supplied in bottles.

Venetoclax should be taken orally once daily until disease progression or unacceptable toxicity is observed. Venetoclax tablets should be taken with a meal and water at approximately the same time each day. Tablets should be swallowed whole and not chewed, crushed, or broken prior to swallowing. If the patient misses a dose within 8 hours of the time it is usually taken, the patient should take the missed dose as soon as possible and resume the normal daily dosing schedule. If a patient misses a dose by more than 8 hours, the patient should not take the missed dose and should resume the usual dosing schedule the next day. If the patient vomits following dosing, no additional dose should be taken that day. The next prescribed dose should be taken at the usual time.

The invention further provides the following non-limiting embodiments:
1. A combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered at least 7 days prior to the first administration of said BCL-2 inhibitor.
2. A combination according to embodiment 1, wherein the anti-CD19 antibody is administered weekly, bi-weekly or monthly after a first administration on Day 1 and wherein the BCL-2 inhibitor is administered for the first time on Day 8.
3. A combination according to any one of the preceding embodiments, wherein the anti-CD19 antibody after a first administration on Day 1 is administered weekly for the first 3 months.
4. A combination according to embodiment 3, wherein the anti-CD19 antibody after a first administration on Day 1 is administered weekly for the first 3 months and bi-weekly for at least the next 3 months.
5. A combination according to embodiment 4, wherein the anti-CD19 antibody after a first administration on Day 1 is administered weekly for the first 3 months, bi-weekly for the next 3 months and monthly thereafter.
6. A combination according to any one of the preceding embodiments, wherein there is an additional dosing of the anti-CD19 antibody on Day 4.
7. A combination according to any one of the preceding embodiments, wherein the BCL-2 inhibitor is administered for the first time on Day 8 with a starting dose of 20mg followed by a weekly ramp-up dosing of 50mg, 100mg, 200mg and 400mg followed by a daily dosing of 400mg.
8. A combination according to any one of the preceding embodiments, wherein said anti-CD19 antibody and said BCL-2 inhibitor are administered separately.
9. A combination according to any one of the preceding embodiments, wherein said BCL-2 inhibitor is venetoclax.
10. A combination according to any one of the preceding embodiments, wherein the anti-CD19 antibody comprises an HCDR1 region comprising sequence SYVMH (SEQ ID NO: 1), an HCDR2 region comprising sequence NPYNDG (SEQ ID NO: 2), an HCDR3 region comprising sequence GTYYYGTRVFDY (SEQ ID NO: 3), an LCDR1 region comprising sequence RSSKSLQNVNGNTYLY (SEQ ID NO: 4), an LCDR2 region comprising sequence RMSNLNS (SEQ ID NO: 5), and an LCDR3 region comprising sequence MQHLEYPIT (SEQ ID NO: 6)
11. A combination according to embodiment 9, wherein the anti-CD19 antibody comprises a variable heavy chain comprising the sequence 
EVQLVESGGGLVKPGGSLKLSCAASGYTFTSYVMHWVRQAPGKGLEWIGYINPYNDGTKY
NEKFQGRVTISSDKSISTAYMELSSLRSEDTAMYYCARGTYYYGTRVFDYWGQGTLVTVSS
(SEQ ID NO: 10)
 and a variable light chain of the sequence 
DIVMTQSPATLSLSPGERATLSCRSSKSLQNVNGNTYLYWFQQKPGQSPQLLIYRMSNLNS
GVPDRFSGSGSGTEFTLTISSLEPEDFAVYYCMQHLEYPITFGAGTKLEIK (SEQ ID NO: 11).
12. A combination according to any of embodiments 9 or 10, wherein the anti-CD19 antibody comprises a heavy chain constant domain comprising the sequence 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPDV
FLFPPKPKDTLMISRTPEVTCVVVDVSHEDPEVQFNWYVDGVEVHNAKTKPREEQFNSTFR
VVSVLTVVHQDWLNGKEYKCKVSNKALPAPEEKTISKTKGQPREPQVYTLPPSREEMTKNQ
VSLTCLVKGFYPSDIAVEWESNGQPENNYKTTPPMLDSDGSFFLYSKLTVDKSRWQQGNV
FSCSVMHEALHNHYTQKSLSLSPGK (SEQ ID NO: 12).
13. A combination according to any of embodiments 10 to 12, wherein the anti-CD19 antibody comprises a light chain constant domain comprising the sequence 

. (SEQ ID NO: 13)
14. A combination according to any of embodiments 10 to 13, wherein the anti-CD19 antibody is administered in a concentration of 12mg/kg.
15. A combination according to any one of the preceding embodiments for use in the treatment of chronic lymphocytic leukemia, small lymphocytic lymphoma or non-Hodgkin's lymphoma, wherein the non-Hodgkin's lymphoma is selected from the group consisting of follicular lymphoma, small lymphocytic lymphoma, mucosa-associated lymphoid tissue, marginal zone, diffuse large B cell, Burkitt's, and mantle cell.

## Claims

1. A combination comprising an anti-CD19 antibody and a BCL-2 inhibitor, for use in the treatment of a patient suffering from non-Hodgkin's lymphoma, chronic lymphocytic leukemia and/or small lymphocytic lymphoma, wherein said anti-CD19 antibody is administered at least 7 days prior to the first administration of said BCL-2 inhibitor.

2. An anti-CD19 antibody for use in combination with a BCL-2 inhibitor in the treatment of a patient suffering from non-Hodgkin's lymphoma, chronic lymphocytic leukemia and/or small lymphocytic lymphoma, wherein said anti-CD19 antibody is administered at least 7 days prior to the first administration of said BCL-2 inhibitor.

3. A BCL-2 inhibitor for use in combination with an anti-CD19 antibody in the treatment of a patient suffering from non-Hodgkin's lymphoma, chronic lymphocytic leukemia and/or small lymphocytic lymphoma, wherein said anti-CD19 antibody is administered at least 7 days prior to the first administration of said BCL-2 inhibitor.

4. The combination for use according to any one of the preceding claims, wherein the anti-CD19 antibody is administered weekly, bi-weekly or monthly after a first administration on Day 1 and wherein the BCL-2 inhibitor is administered for the first time on Day 8, optionally wherein the BCL-2 inhibitor is administered with a starting dose for a weekly ramp-up.

5. The combination for use according to any one of the preceding claims, wherein the anti-CD19 antibody after a first administration on Day 1 is administered weekly for the first 3 months.

6. The combination for use according to claim 5, wherein the anti-CD19 antibody after a first administration on Day 1 is administered weekly for the first 3 months and biweekly for at least the next 3 months.

7. The combination for use according to claim 6, wherein the anti-CD19 antibody after a first administration on Day 1 is administered weekly for the first 3 months, bi-weekly for the next 3 months and monthly thereafter.

8. The combination for use according to any one of the preceding claims, wherein there is an additional dosing of the anti-CD19 antibody on Day 4.

9. The combination for use according to any one of the preceding claims, wherein the BCL-2 inhibitor is administered for the first time on Day 8 with a starting dose of 20mg followed by a weekly ramp-up dosing of 50mg, 100mg, 200mg and 400mg, optionally followed by a daily dosing of 400mg.

10. The combination for use according to any one of the preceding claims, wherein said anti-CD19 antibody and said BCL-2 inhibitor are formulated in different pharmaceutical compositions.

11. The combination for use according to any one of the preceding claims, wherein said anti-CD19 antibody and said BCL-2 inhibitor are administered separately.

12. The combination for use according to any one of the preceding claims, wherein said anti-CD19 antibody and said BCL-2 inhibitor are administered at a time where both components of the combination are active in the patient at the same time.

13. The combination for use according to any one of the preceding claims, wherein said BCL-2 inhibitor is venetoclax.

14. The combination for use according to any one of the preceding claims, wherein the anti-CD19 antibody comprises an HCDR1 region comprising sequence SYVMH (SEQ ID NO: 1), an HCDR2 region comprising sequence NPYNDG (SEQ ID NO: 2), an HCDR3 region comprising sequence GTYYYGTRVFDY (SEQ ID NO: 3), an LCDR1 region comprising sequence RSSKSLQNVNGNTYLY (SEQ ID NO: 4), an LCDR2 region comprising sequence RMSNLNS (SEQ ID NO: 5), and an LCDR3 region comprising sequence MQHLEYPIT (SEQ ID NO: 6)

15. The combination for use according to any one of the preceding claims, wherein the anti-CD19 antibody comprises a variable heavy chain comprising the sequence 
EVQLVESGGGLVKPGGSLKLSCAASGYTFTSYVMHWVRQAPGKGLEWIGYINPYNDG
TKYNEKFQGRVTISSDKSISTAYMELSSLRSEDTAMYYCARGTYYYGTRVFDYWGQG
TLVTVSS
(SEQ ID NO: 10)
 and a variable light chain of the sequence

DIVMTQSPATLSLSPGERATLSCRSSKSLQNVNGNTYLYWFQQKPGQSPQLLIYRMSN
LNSGVPDRFSGSGSGTEFTLTISSLEPEDFAVYYCMQHLEYPITFGAGTKLEIK (SEQ ID
NO: 11).

16. The combination for use according to any one of the preceding claims, wherein the anti-CD19 antibody comprises a heavy chain constant domain comprising the sequence

ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPE
LLGGPDVFLFPPKPKDTLMISRTPEVTCVVVDVSHEDPEVQFNWYVDGVEVHNAKTKP
REEQFNSTFRVVSVLTVVHQDWLNGKEYKCKVSNKALPAPEEKTISKTKGQPREPQV
YTLPPSREEMTKNQVSLTCLVKGFYPSDIAVEWESNGQPENNYKTTPPMLDSDGSFFL
YSKLTVDKSRWQQGNVFSCSVMHEALHNHYTQKSLSLSPGK (SEQ ID NO: 12).

17. The combination for use according to any one of the preceding claims, wherein the anti-CD19 antibody comprises a light chain constant domain comprising the sequence


 (SEQ ID
NO: 13).

18. The combination for use according to any one of claims 14 to 17, wherein the anti-CD19 antibody is administered in a concentration of 12 mg/kg.

19. The combination for use according to any one of the preceding claims for use in the treatment of chronic lymphocytic leukemia, small lymphocytic lymphoma or non-Hodgkin's lymphoma, wherein the non-Hodgkin's lymphoma is selected from the group consisting of follicular lymphoma, small lymphocytic lymphoma, mucosa-associated lymphoid tissue lymphoma, marginal zone lymphoma, diffuse large B cell lymphoma, Burkitt's lymphoma, and mantle cell lymphoma.
